# EUROPEAN PATENT SPECIFICATION

(11) **EP 4 463 128 B1**
(45) Date of publication and mention of the grant of the patent: **05.11.2025**
(21) Application number: 23700030.2
(22) Date of filing: 09.01.2023
(51) Int. Cl.: A61K 8/04, A61K 8/38, A61K 8/67, A61Q 11/00, A61K 8/24

(54) **TEETH WHITENING COMPOSITION WITH VITAMINS**
ZAHNBLEICHZUSAMMENSETZUNG MIT VITAMINEN
COMPOSITION DE BLANCHIMENT DES DENTS CONTENANT DES VITAMINES

(30) Priority: 13.01.2022 IT 202200000479
(43) Date of publication of application: 20.11.2024
(73) Proprietor: IDS RESEARCH SOCIETÀ A RESPONSABILITÀ LIMITATA, 16122 Genova (IT)
(72) Inventor: PIERGALLINI, Remigio, 63066 GROTTAMMARE (AP) (IT); LOUPIS, Nikolaos, 15231 Chalandri (GR)
(74) Representative: Cutropia, Gianluigi
(86) International application number: PCT/IB2023/050162
(87) International publication number: WO 2023/135503

(56) References cited:
- WO-A1-2007/066837
- WO-A2-2011/161238
- US-A1- 2021 069 096

## Description

The present invention relates to a teeth whitening composition that contains vitamins, as defined in claim 1.

Peroxides are generally used for the whitening of human teeth in the form of gels that can be applied at home with a customized tray for overnight use or in the dental practice where they are applied by the operators of the dental practice directly on the tooth surface or in a dental alignment tray.

The peroxides of the compositions are gradually decomposed, releasing molecular oxygen and active forms of oxygen. The active forms normally undergo a spontaneous degradation, but they are not abundant due to the action of the antioxidant enzymes of the saliva and of the gingival crevicular fluid.

The ingredients of the teeth whitening composition used in the dental practice must be more aggressive in order to release more active forms of oxygen in a shorter time to immediately achieve an acceptable whitening result in a dental session of medium duration. In order to accomplish such a purpose, the ingredients are generally activated by means of physical or chemical activators.

The chemical activators that are typically used are metal ions, such as iron ions, or other types of chemical activators, such as alkaline solutions mixed with a peroxide gel. In an alkaline environment, the peroxides are decomposed more rapidly, generating oxygen, even if the presence of radical forms of oxygen is limited.

It has been noted in the prior art that the active forms of oxygen are much more effective for teeth whitening. The more radicals are produced, the better the teeth whitening effect will be.

Photoabsorbent molecules, such as biologically acceptable coloring agents, are used to intensify the teeth whitening effect and amplify the production of active oxygen.

Certain colors selectively absorb different wavelengths of the light emitted by LEDs or lasers and in such a way they can store energy, which is successively released in the environment and also in the peroxides contained in the compositions, catalyzing the production of active forms of oxygen.

It has been proven that the luminous energy can intensify the production of free radicals.

Having a high concentration of peroxides, the teeth whitening compositions of the prior art tend to damage the dental enamel and are poorly tolerated by the exposed dentin of the teeth and by the open dentinal tubules. Moreover, the prolonged presence of a gel containing hydrophilic or hygroscopic molecules in contact with the teeth will cause a dehydration of the enamel and of the dentin, which can cause pulpal hyperemia.

For such a reason, in order to whiten the teeth during a session with dental apparatuses, recently, it has been necessary to use whitening materials that are less aggressive and possibly protective or even capable of mitigating the discomfort caused by the forces exerted on the teeth by the dental apparatuses.

WO2011161238A2 discloses a tooth remineralizing composition comprising an aqueous solution containing sodium hydroxide or potassium hydroxide, fluoride salt, hydroxyapatite and xylitol.

The purpose of the present invention is to eliminate the drawbacks of the prior art by providing a composition for dental use that is efficient and effective in the whitening of teeth, and at the same time minimally aggressive and protective.

Another purpose is to provide such a composition for dental use that is safe, reliable and suitable for a protection of the dental enamel and a desensitization of the exposed dentin.

Another purpose is to provide a teeth whitening composition that can be easily applied by the dentist and by the end consumer.

These purposes are achieved in accordance with the invention with the features of the attached independent claim 1.

Advantageous realizations of the invention appear from the dependent claims.

The composition according to the invention is defined in claim 1.

The synergistic combination of the vitamins D and K with the peroxides and the hydroxyapatite molecules of the composition generates an amplified protective action on the teeth, making the composition suitable for teeth whitening, enamel protection and dentin desensitization of the teeth treated with such a composition. Thus, the composition according to the invention has a compensatory effect on the irritating effect of the peroxide and of the dehydration caused by the gel.

It is generally known that in the human body the vitamins D and K participate in mechanisms for the restructuring of the bone tissue at all stages of life.

The composition according to the invention creates a regenerative environment based on the synergistic mechanisms between the vitamins D and K and the peroxides of the composition in order to structurally protect and repair the dental tissues (enamel and dentin) that are exposed to the peroxides of the whitening materials of the composition. The peroxide-based whitening materials come into prolonged contact with the surface of the enamel and of the exposed dentin for the purpose of oxidizing the organic residues that have penetrated into the structure of the surface of the teeth in order to clean the surface and make the teeth appear whiter.

In the composition according to the invention, molecules of vitamins D and vitamins K are incorporated into the peroxide-based whitening materials, with a strongly synergistic action, resulting in a compensation of such oxidation of the dental tissues and of the dental sensitivity during and after the whitening treatment.

Additional features of the invention will appear clearer from the following detailed description, which refers to its merely illustrative and therefore non-limiting embodiments, illustrated in the attached examples and drawings, wherein:
Fig. 1 is a schematic sectional view of a portion of a tooth illustrating the application of the composition according to the invention with a dental tray; and
Fig. 2 is an enlarged detail of Fig. 1, illustrating the chemical reactions in the composition according to the invention.

The composition according to the invention comprises a first gel comprising a peroxide and a second gel comprising vitamin K, vitamin D and hydroxyapatite (Ca₁₀(PO₄)₆ (OH)₂).

The two gels must remain separate and must be mixed before being applied on the patient's teeth in order to obtain the final composition.

The preparation of the two gels is basically identical. In both cases, a solution of water and glycerin with a gelling agent, such as Carbopol, is used, and then the other components are gradually added with mechanical mixing, until a homogeneous gel is obtained.

A pH regulator, such as ethylenediaminetetraacetic acid (EDTA), anhydrous disodium phosphate (Na₂HPO₄) and/or sodium hydroxide (NaOH), is added to both gels to achieve a desired pH.

The first gel must have a pH less than or equal to 5. The pH of the first gel cannot be higher than 5, because it would result in the decomposition of the peroxide molecule inside the package. Preferably, the pH of the first gel is comprised between 4.5 and 5.

On the other hand, the pH of the second gel can have a value higher than 6, in such a way that the final composition is brought to a pH value of 6, which is well tolerated by both the enamel and the dentin. By way of example, the pH of the second gel is comprised between 6.5 and 7.

In the first gel, carbamide peroxide is preferably used as peroxide, it being preferred over the hydrogen peroxide because it provides a gradual release of oxygen. In such a way, the protective action of the vitamin complexes and of the hydroxyapatite contained in the final composition can be efficacious for weeks or even months of application.

In the second gel, a vitamin D comprising one or more groups of the following fat-soluble pro-hormones comprising one or more of the following vitamins D1, D2, D3, D4 and D5 is used. Preferably, vitamin D3 (cholecalciferol) and/or vitamin D2 (ergocalciferol) is used.

The vitamin K used in the second gel preferably comprises vitamin K2 (menaquinone of bacterial origin), and/or vitamin K3 (water-soluble menadione of synthetic origin).

The hydroxyapatite of the second gel is a ground hydroxyapatite with nanometric particles.

The second gel may also comprise a solvent, such as phenoxyethanol (C₈H₁₀O₂).

The two gels are mixed together in such a way to obtain the final composition.

Advantageously, the final composition contains peroxide in a weight percentage comprised between 6% and 8% relative to the weight of the final composition. Such an amount of peroxide is lower than the amount of peroxide used in the compositions of the prior art.

For such a reason, the composition according to the invention is suitable for teeth whitening for a long-term use. The composition can be inserted in dental alignment trays, or it can be used in teeth whitening trays by patients with dental sensitivity and exposed dentin who choose a less aggressive whitening treatment.

The low peroxide content makes the composition much less aggressive and also less effective, in terms of whitening effect, than the compositions with a high peroxide content of the prior art. Thus, the composition according to the invention requires longer application times than the compositions of the prior art.

In the final composition:
the vitamin K has a weight percentage comprised between 0.25 and 0.35 relative to the weight of the final composition;
the vitamin D has a weight percentage comprised between 0.25 and 0.35 relative to the weight of the final composition; and
the hydroxyapatite has a weight percentage comprised between 0.7 and 0.9 relative to the weight of the final composition.

Two examples of the composition are shown below.

### EXAMPLE 1

| 1^{st} GEL | WEIGHT % |
|---|---|
| Carbamide Peroxide | 8.75 |
| Carbopol 980NF | 1.50 |
| EDTA | 0.20 |
| Glycerin | 60.00 |
| Purified water | 29.45 |
| Disodium Phosphate Anhydrous | 0.10 |

The first gel has a pH of 4.5-5.

| 2^{nd} GEL | WEIGHT % |
|---|---|
| Carbopol 980NF | 1.80 |
| EDTA | 0.20 |
| Glycerin | 50.00 |
| Purified water | 40.50 |
| Disodium Phosphate Anhydrous | 0.50 |
| Vitamin K2 in powder form | 1.50 |
| Vitamin D3 in powder form | 1.50 |
| Nanometric hydroxyapatite | 4 |

The second gel has a pH of 6.5-7.

In such a case, the mixing ratio between the first gel and the second gel is 4:1.

### EXAMPLE 2

| 1^{st} GEL | WEIGHT % |
|---|---|
| Carbamide Peroxide | 14.00 |
| Carbopol 980NF | 1.50 |
| EDTA | 0.20 |
| Glycerin | 60.00 |
| Purified water | 24.25 |
| Disodium Phosphate Anhydrous | 0.10 |

The first gel has a pH of 4.5-5.

| 2^{nd} GEL | % WEIGHT |
|---|---|
| Carbopol 980NF | 1,80 |
| EDTA | 0,20 |
| Glycerin | 50,00 |
| Purified water | 44,20 |
| Disodium Phosphate Anhydrous | 0,50 |
| Vitamin K2 powder | 0,60 |
| Vitamin D3 powder | 0,60 |
| Nanometric hydroxyapatite | 1,60 |
| Phenoxyethanol | 0,50 |

The first gel has a PH of 6.5-7.

In such a case, the mixing ratio between the first gel and the second gel is 1:1.

The two gels are packaged in a kit comprising two syringes filled with the two gels, respectively. The two syringes are connected with a suitable connector and the gel contained in a syringe is injected into the other syringe so that the two gels are mixed. The final product is contained in one of the two syringes and is applied in an orthodontic tray that is placed in the mouth by the patient (posterior and lower arch) and kept for approximately 8 hours (usually overnight).

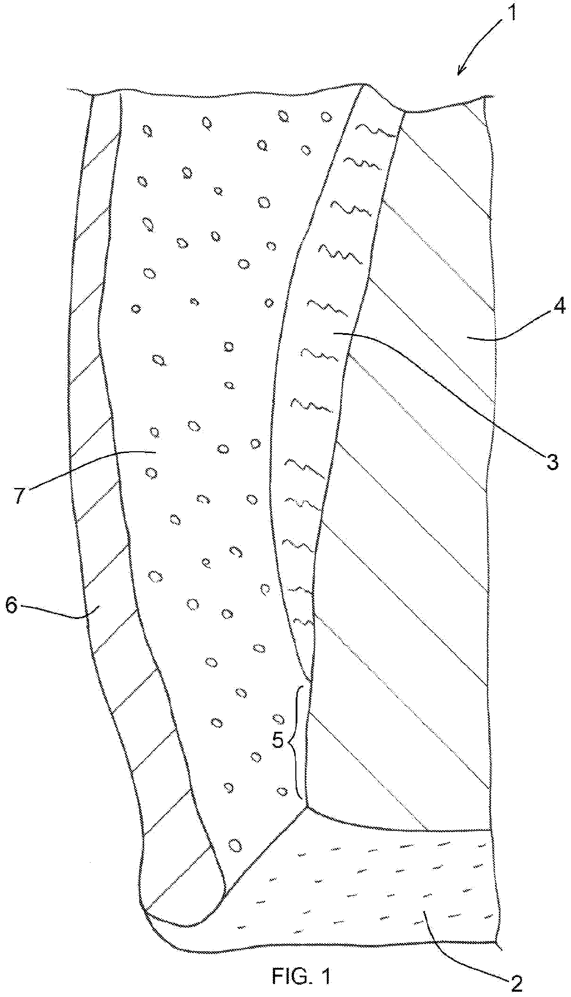

Fig. 1 illustrates a portion of a tooth (1) disposed on a gum (2). The tooth (1) has enamel (3) that covers the dentin (4). A portion of exposed dentin (5) near the gum (2) is not covered by the enamel (3) and is therefore a sensitive portion of the tooth.

A composition (7) according to the invention is applied in a dental tray (6) that is disposed in the patient's mouth, so that the composition goes into contact (7) with the enamel (3) of the tooth and with the exposed dentin (5) that is not covered by the enamel (3).

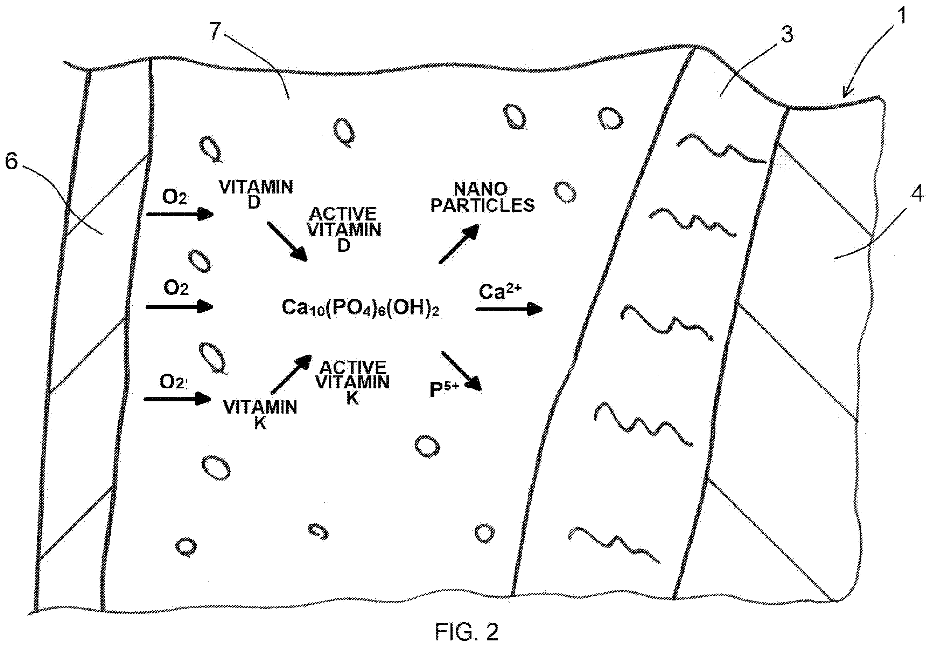

With reference to Fig. 2, in the present invention, the oxidative action of the peroxide that produces oxygen (O₂) is used to trigger the activation mechanism of the vitamin D and K complexes and of the structural hydroxyapatite molecules (Ca₁₀(PO₄)₆(OH)₂) embedded in the composition, in order to produce calcium ions (Ca²⁺), phosphorus ions (P⁵⁺) and an amplified flow of hydroxyapatite nanoparticles that adhere to the surface of the enamel (3) and of the exposed dentin (5).

Generally speaking, vitamin D is in a biologically inactive form and must undergo hydroxylation reactions in order to be transformed into a biologically active form consisting of calcitriol.

The hydroxylation of the vitamin D can be obtained on different substrates in several chemical ways. In the case of the composition according to the invention, the hydroxylation of the vitamin D is obtained in a radical way by means of reactive forms of the oxygen released by the peroxides contained in the composition.

The basic biochemical action of the vitamin K is the g- carboxylation of the glutamine residues of various proteins. Such an action is done with the oxidation of the vitamin K, and the necessary energy that allows the binding of the vitamin K with the calcium ions (Ca²⁺) is released.

A process of continuous demineralization and remineralization occurs at the level of the enamel (3) and of the exposed dentin (5), with the loss and the reacquisition of calcium ions and phosphorus ions which belong to the hydroxyapatite structure of the dentin.

The use of nanometric hydroxyapatite in the composition, as a source of calcium ions and phosphorus ions and as a source of nano-particles of its own structure and of chondroitin-sulfate A and B, with catalyzing action of the vitamins D and K, liberates a forced deposition process of the ions and of the structural nano-particles of the hydroxyapatite in the microfractures of the enamel (3) and in the dentinal tubules of the exposed dentin (5).

In addition, there is a stimulated flow of amplified production of molecules suitable for polarizing the vitamins D and K, which is obtained by means of the contact of the peroxide with the biofilm and the enzymes of the saliva (SOD/Catalase enzyme mechanism), causing a polarization of the vitamins D and K, which become active.

The reactions that take place in the composition applied and maintained on the dental tissues for extended periods of time result in an amplified oxidation of the pigments and the organic macromolecules that have penetrated the enamel (3) and in a reinforced flow of calcium ions and phosphorus ions created by the vitamin complexes and by the hydroxyapatite nanoparticles.

According to the micro-environmental conditions created by the composition according to the invention, surprisingly, a dual action of forced oxidation of the enamel (3) and of simultaneous protection and repair of the exposed dentin (5) is observed, with a consequent reduction of the sensitivity of the teeth during and after the treatment.

At the level of dental enamel, the spaces devoid of the pigments and the organic residues are the site of stimulated and forced deposition of nanometric hydroxyapatite nanoparticles, of calcium ions and phosphorus ions due to the polarizing effect of the vitamin complexes activated by the oxygen generated by the peroxides.

Before being applied, the composition according to the invention is stable, and the stability of its components was proven by means of special aging tests done in a laboratory.

When the composition comes into contact with the surface of the tooth to be whitened, the decomposition of the peroxides is amplified due to the effect of the oral anti-oxidant enzymes, with consequent release of oxygen and of its reactive forms and catalyzation of the process as described above.

Surprisingly, the experimental clinical cases showed that the patients did not experience any dental sensitivity to the composition according to the invention, and, on the contrary, even in the cases in which the teeth were very sensitive to thermal stimuli or pressure before the application of the composition, such a sensitivity decreased or even disappeared after the application of the composition.

## Claims

1. Kit comprising two syringes filled with two gels, wherein a syringe is filled with a first gel comprising a peroxide, and the other syringe is filled with a second gel comprising vitamin K, vitamin D, and hydroxyapatite; wherein said hydroxyapatite of the second gel is a ground hydroxyapatite with nanometric particles,
the two syringes being connected and the gel contained in a syringe being injected into the other syringe so that the two gels are mixed together to obtain a final teeth whitening composition contained in one of the two syringes.

2. Kit according to claim 1, wherein the first gel comprises a pH regulator in order to have a pH lower than or equal to 5, preferably comprised between 4.5 and 5.

3. Kit according to claim 2, wherein said pH regulator of the first gel comprises ethylenediaminetetraacetic acid (EDTA), disodium hydrogen phosphate (Na₂HPO₄) and/or sodium hydroxide (NaOH).

4. Kit according to any one of the preceding claims, wherein the second gel comprises a pH regulator in order to have a pH higher than 6, preferably comprised between 6.5 and 7.

5. Kit according to claim 4, wherein said pH regulator of the second gel comprises ethylenediaminetetraacetic acid (EDTA), disodium hydrogen phosphate (Na₂HPO₄) and/or sodium hydroxide (NaOH).

6. Kit according to any one of the preceding claims, wherein said vitamin D of the second gel comprises vitamin D3 (cholecalciferol) and/or vitamin D2 (ergocalciferol).

7. Kit according to any one of the preceding claims, wherein said vitamin K of the second gel comprises vitamin K2 (menaquinone of bacterial origin), and/or vitamin K3 (water-soluble menadione of synthetic origin).

8. Kit according to any one of the preceding claims, wherein said peroxide of the first gel comprises carbamide peroxide.

9. Kit according to any one of the preceding claims, wherein in said final composition the peroxide has a weight percentage comprised between 6% and 8% relative to the weight of the final composition.

10. Kit according to any one of the preceding claims, wherein in said final composition:
the vitamin K has a weight percentage comprised between 0.25 and 0.35 relative to the weight of the final composition;
the vitamin D has a weight percentage comprised between 0.25 and 0.35 relative to the weight of the final composition; and
the hydroxyapatite has a weight percentage comprised between 0.7 and 0.9 relative to the weight of the final composition.

## Patentansprüche

1. Kit, umfassend zwei mit Gelen gefüllte Spritzen, wobei eine Spritze mit einem ersten Gel, umfassend ein Peroxid, gefüllt ist, und die andere Spritze mit einem zweiten Gel, umfassend Vitamin K, Vitamin D und Hydroxylapatit gefüllt ist; wobei das Hydroxylapatit des zweiten Gels ein gemahlenes Hydroxylapatit mit nanometrischen Partikeln ist,
wobei die beiden Spritzen verbunden sind und das in einer Spritze enthaltene Gel in die andere Spritze eingespritzt wird, so dass die beiden Gele miteinander vermischt werden, um eine fertige Zahnaufhellungszusammensetzung in einer der beiden Spritzen zu erhalten.

2. Kit nach Anspruch 1, wobei das erste Gel einen pH-Regulator umfasst, um einen pH-Wert kleiner oder gleich 5, vorzugsweise zwischen 4,5 und 5 zu erhalten.

3. Kit nach Anspruch 2, wobei der pH-Regulator des ersten Gels Ethylendiamintetraessigsäure (EDTA), Dinatriumhydrogenphosphat (Na₂HPO₄) und/oder Natriumhydroxid (NaOH) umfasst.

4. Kit nach einem der vorstehenden Ansprüche, wobei das zweite Gel einen pH-Regulator umfasst, um einen pH-Wert größer 6, vorzugsweise zwischen 6,5 und 7 zu erhalten.

5. Kit nach Anspruch 4, wobei der pH-Regulator des zweiten Gels Ethylendiamintetraessigsäure (EDTA), Dinatriumhydrogenphosphat (Na₂HPO₄) und/oder Natriumhydroxid (NaOH) umfasst.

6. Kit nach einem der vorstehenden Ansprüche, wobei das Vitamin D des zweiten Gels Vitamin D3 (Cholecalciferol) und/oder Vitamin D2 (Ergocalciferol) umfasst.

7. Kit nach einem der vorstehenden Ansprüche, wobei das Vitamin K des zweiten Gels Vitamin K2 (Menachinon bakteriellen Ursprungs) und/oder Vitamin K3 (wasserlösliches, synthetisch hergestelltes Menadion) umfasst.

8. Kit nach einem der vorstehenden Ansprüche, wobei das Peroxid des ersten Gels Carbamidperoxid umfasst.

9. Kit nach einem der vorstehenden Ansprüche, wobei das Peroxid in der fertigen Zusammensetzung einen Gewichtsprozentsatz im Bereich von 6 bis 8 % in Bezug auf das Gewicht der fertigen Zusammensetzung aufweist.

10. Bausatz nach einem der vorstehenden Ansprüche, wobei in der fertigen Zusammensetzung:
das Vitamin K einen Gewichtsprozentsatz im Bereich von 0,25 bis 0,35 in Bezug auf das Gewicht der fertigen Zusammensetzung aufweist;
das Vitamin D einen Gewichtsprozentsatz im Bereich von 0,25 bis 0,35 in Bezug auf das Gewicht der fertigen Zusammensetzung aufweist; und
das Hydroxylapatit einen Gewichtsprozentsatz im Bereich von 0,7 bis 0,9 in Bezug auf das Gewicht der fertigen Zusammensetzung aufweist.

## Revendications

1. Kit comprenant deux seringues remplies avec deux gels, où une seringue est remplie avec un premier gel comprenant un peroxyde et l'autre seringue est remplie avec un second gel comprenant de la vitamine K, de la vitamine D et de l'hydroxyapatite : où ladite hydroxyapatite du second gel est une hydroxyapatite moulue avec des particules de dimensions nanométriques,
les deux seringues étant reliées et le gel contenu dans une seringue étant injecté dans l'autre seringue, de manière que les deux gels soient mélangés entre eux pour obtenir une composition finale, pour le blanchiment dentaire, contenue dans l'une des deux seringues.

2. Kit selon la revendication 1, où le premier gel comprend un régulateur de pH pour avoir un pH mineur ou égal à 5, préférablement compris entre 4,5-5.

3. Kit selon la revendication 2, où ledit régulateur de pH du premier gel comprend de l'acide éthylène diamine tétraacétique (EDTA), du monohydrogéno-orthophosphate de sodium (Na₂HPO₄) et/ou de l'hydroxyde de sodium (NaOH).

4. Kit selon l'une quelconque des revendications précédentes, où le second gel comprend un régulateur de pH pour avoir un pH majeur de 6, préférablement compris entre 6,5-7.

5. Kit selon la revendication 4, où ledit régulateur de pH du second gel comprend de l'acide éthylène diamine tétraacétique (EDTA), du monohydrogéno-orthophosphate de sodium (Na₂HPO₄) et/ou de l'hydroxyde de sodium (NaOH).

6. Kit selon l'une quelconque des revendications précédentes, où ladite vitamine D du second gel comprend de la vitamine D3 (cholécalciférol) et/ou de la vitamine D2 (ergocalciférol).

7. Kit selon l'une quelconque des revendications précédentes, où ladite vitamine K du second gel comprend de la vitamine K2 (ménaquinone d'origine bactérienne), et/ou de la vitamine K3 (ménadione hydrosoluble d'origine synthétique).

8. Kit selon l'une quelconque des revendications précédentes, où ledit peroxyde du premier gel comprend du peroxyde de carbamide.

9. Kit selon l'une quelconque des revendications précédentes, où dans ladite composition finale, le peroxyde a un pourcentage poids compris entre 6-8% par rapport au poids de la composition finale.

10. Kit selon l'une quelconque des revendications précédentes, où dans ladite composition finale :
la vitamine K a un pourcentage poids compris entre 0,25-0,35 par rapport au poids de la composition finale ;
la vitamine D a un pourcentage poids compris entre 0,25-0,35 par rapport au poids de la composition finale ; et
l'hydroxyapatite a un pourcentage poids compris entre 0,7-0,9 par rapport au poids de la composition finale.
